# EUROPÄISCHE PATENTSCHRIFT

(11) **EP 1 366 742 B1**
(45) Veröffentlichungstag und Bekanntmachung des Hinweises auf die Patenterteilung: **10.04.2013**
(21) Anmeldenummer: 03011976.2
(22) Anmeldetag: 28.05.2003
(51) Int. Cl.: A61K 8/73, A61Q 5/02, A61Q 5/12

(54) **Mildes Pflegeshampoo quaternisierte Guar- und Cellulosederivate enthaltend**
Mild hair conditioning shampoo comprising quaternised guar and cellulose derivatives
Shampooing conditionnant doux contenant des dérivés de guar et de cellulose quaternisés

(30) Priorität: 31.05.2002 DE 10224022
(43) Veröffentlichungstag der Anmeldung: 03.12.2003
(73) Patentinhaber: Beiersdorf AG, 20245 Hamburg (DE)
(72) Erfinder: Demitz, Michael, 22529 Hamburg (DE); Argembeaux, Horst, 21465 Wentorf (DE); Albrecht, Harald, 22083 Hamburg (DE); Heitmann, Birgit, 22769 Hamburg (DE); Primmel, Bettina, 20146 Hamburg (DE); Kaiser, Elke, 22607 Hamburg (DE)

(56) Entgegenhaltungen:
- WO-A-00/66081
- WO-A-97/26860
- WO-A-98/50007
- WO-A-99/38475
- HOESSEL P ET AL: "CONDITIONING POLYMERS IN TODAY'S SHAMPOO FORMULATIONS - EFFICACY, MECHANISM AND TEST METHODS" INTERNATIONAL JOURNAL OF COSMETIC SCIENCE, KLUWER ACADEMIC PUBLISHERS, DORDRECHT, NL, Bd. 22, Nr. 1, 2000, Seiten 1-10, XP001154382 ISSN: 0142-5463
- PATEL, C.U.: "Anti-static properties of some cationic polymers used in hair care products" INTERNATIONAL JOURNAL OF COSMETIC SCIENCE, Bd. 5, Nr. 5, - 1983 Seiten 181-188, XP008021180
- BUSCH, P ET AL: "Objektive Bestimmung von Avivage-Effekten am Humanhaar" SEIFEN-ÖLE-FETTE-WACHSE, Bd. 108, Nr. 10, - 16. Juni 1982 (1982-06-16) XP002252497

## Beschreibung

Die vorliegende Erfindung betrifft eine kosmetische Zubereitung enthaltend ein oder mehrere Guar-Hydroxypropyltrimethylammoniumchloride, ein oder mehrere Polymere, welche gebildet werden aus quaternisierten Ammoniumsalz der Hydroxypropylcellulose, die mit einem Trimethylammonium substituierten Epoxid modifiziert ist (INCI: Polyquaternium-10), sowie ein oder mehrere Tenside.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurück ließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:

Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycolmonolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen, in der Regel kationische Polymere, stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Ein Nachteil am Stande der Technik besteht in dem Umstand, dass durch häufiges Waschen oder chemische Behandlung (Bleichen, Färben) das Haar stark geschädigt wird. Insbesondere führt die Entfernung/Zerstörung von natürlich im Haar vorkommenden Fettkomponenten zu einem Verlust an Glätte, Geschmeidigkeit und Glanz. Das Haar erscheint dann trocken und spröde. Um diese Schäden ausgleichen zu können, werden den Haarpflegeprodukten in der Regel große Mengen an kationischen Polymeren als Haarkonditionierem zugesetzt. Um den Fettverlust auszugleichen, werden derartigen Produkten häufig Silikonverbindungen oder weitere hydrophobe Konditionierer zugesetzt. Diese nach dem Stande der Technik hergestellten Produkte haben jedoch eine Reihe von Nachteilen:
■ Es lagern sich bei jeder Anwendung herkömlicher Produkte zusätzliche Schichten an Konditionierer auf dem Haar ab (engl. build-up-effect). Das Haar wird durch die großen Mengen an Konditionierern beschwert und bekommt ein strähniges Aussehen.
■ Die Produkte enthalten wasserlösliche und fettlösliche Bestandteile, die zu einer Emulsion dispergiert sind. Die Herstellung solcher Emulsionen ist jedoch sehr aufwendig. Es kann dabei immer wieder zu Unverträglichkeiten von einzelnen Inhaltsstoffen (Aufrahmen der Fettphase, Ausfällungen) kommen.
■ Produkte aus Emulsionsbasis besitzen meist ein milchig-trübes Aussehen. Diese lassen sich nur unzureichend mit optischen Effekten (Farbschlieren, Blasen, farbige Wirkstoffkügelchen, Glitterstoffe) attraktiver gestalten.

Es war daher die Aufgabe der vorliegenden Erfindung die Mängel des Standes der Technik zu beseitigen oder zumindest zu lindern und gut verträgliche Haarpflegeprodukte zu entwickeln, welche selbst stark geschädigtes Haar wieder zu neuem Glanz, Fülle und Spannkraft verhelfen. Ferner war es die Aufgabe der vorliegenden Erfindung, eine auf wasserlöslichen Bestandteilen zusammengesetzte transparente Zubereitung zu entwickeln.

Überraschend gelöst wird die Aufgabe durch eine kosmetische Zubereitung enthaltend
a) ein oder mehrere Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen,
b) ein oder mehrere Polymere, welche gebildet werden aus quaternisierten Ammoniumsalz der Hydroxypropylcellulose die mit einem Trimethylammonium substituierten Epoxid modifiziert sind (INCI: Polyquaternium-10) und ein mittleres Molekulargewicht von 200000 bis 1000000 und eine Ladungsdichte von 0,8 bis 1,8 meq/g aufweisen,
c) Natriumlaurylethersulfat,
d) Trübungsmittel/Perlglänze gewählt aus PEG-3 Distearat und/oder eine Kombination aus Glycoldistearat, Glycerin, Laureth-4 und Cocamidopropylbetain und/oder eine Kombination aus Glycoldistearat, Cocosglucosiden, Glycoryloleat und Glycerylstearat und/oder
   Styrol/Acrylat Copolymere
   neben gegebenenfalls weiteren kosmetischen Wirk-, Hilfs- und Zusatzstoffen.

Zwar kennt der Stand der Technik die WO 97/26860, WO 99/38475, WO 98/50007 und WO 00/66081 doch konnten diese Schriften nicht den Weg zur vorliegenden Erfindung weisen.

Die erfindungsgemäßen Zubereitungen zeichnen sich dadurch aus, dass die übermäßige Anreicherung von Konditionierern auf dem Haar vermieden wird und das Haar Glanz, Spannkraft und Fülle erhält, ohne strähnig zu wirken. Die erfindungsgemäßen Zubereitungen lassen sich, obwohl sie als Emulsion vorliegen, ungewöhnlich einfach formulieren und weisen ein transparentes Aussehen auf.

Es ist dabei erfindungsgemäß von Vorteil, wenn ein oder mehrere Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen, in einer Konzentration von 0,01 bis 3 Gewichts-%, bevorzugt in einer Konzentration von 0,05 bis 2,5 Gewichts-% und ganz besonders bevorzugt in einer Konzentration von 0,08 bis 2 Gewichts-%, jeweils bezogen auf das Gesamtgewicht der Zubereitung in der Zubereitung enthalten ist.

Erfindungsgemäß vorteilhaft werden die erfindungsgemäßen Guar-Hydroxypropyltrimethylammoniumchloride aus der Jaguar-Serie gewählt, wobei Jaguar Excel der Firma Rhodia erfindungsgemäß ganz besonders bevorzugt ist.

Es ist erfindungsgemäß vorteilhaft, wenn der oder die Polymere, welche gebildet werden aus quaternisierten Ammoniumsalz der Hydroxypropylcellulose die mit einem Trimethylammonium substituierten Epoxid modifiziert ist (INCI: Polyquaternium-10), ein mittleres Molekulargewicht von 200000 bis 1000000 und eine Ladungsdichte von 0,8 bis 1,8 meq/g aufweisen. Erfindungsgemäß vorteilhaft werden diese Polymere in einer Konzentration von 0,01 bis 3 Gewichts-%, bevorzugt in einer Konzentration von 0,05 bis 2,5 Gewichts-% und ganz besonders bevorzugt in einer Konzentration von 0,08 bis 2,0 Gewichts-%, jeweils bezogen auf das Gesamtgewicht der Zubereitung eingesetzt. Das erfindungsgemäß besonders bevorzugte Polymer, welches gebildet wird aus quaternisiertem Ammoniumsalz der Hydroxypropylcellulose die mit einem Trimethylammonium substituierten Epoxid modifiziert ist, stellt das Ucare Polymer JR 400 der Firma Amerchol dar.

Das Dokument WO97/26860 beschreibt kosmetische Zubereitungen enthaltend:
a) ein Guar-Hydroxypropyltrimethylammoniumchlorid,
b) ein Polyquaternium-10,
c) mehrere Tenside und
d) Trübungsmittel/ Perlglänze.

Es ist erfindungsgemäß von Vorteil, wenn das Verhältnis der Komponenten a) und b) von 0,1:10 bis 10:0,1, bevorzugt von 1:10 bis 10:1 , und ganz besonders bevorzugt von 1:5 bis 5:1 beträgt.

Die erfindungsgemäßen kosmetischen Zubereitungen enthalten Tenside. Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl-oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Cocoamidopropylbetain und/oder Dinatrium PEG-5 Laurylcitratsulfosuccinat und/oder N-Kokosfettsäureamidoethyl-N-hydroxyethylglycinat Natriumsalze eingesetzt werden.

Ferner können Polysorbate als waschaktive Agentien erfindungsgemäß vorteilhaft in die Emulsion eingearbeitet werden.

Es ist erfindungsgemäß von Vorteil, wenn ein oder mehrere Tenside in einer Konzentration von 1 bis 25 Gewichts-%, bevorzugt in einer Konzentration 7 von 20 bis Gewichts-% und ganz besonders bevorzugt in einer Konzentration von 8 bis 18 Gewichts-%, jeweils bezogen auf das Gesamtgewicht der Zubereitung eingesetzt werden.

Ferner ist es erfindungsgemäß von Vorteil, wenn in den erfindungsgemäßen Zubereitungen Salze eingesetzt werden. Diese führen zu einer Verdickung der Zubereitung, so dass Zubereitungen mit Viskositäten von 2000 mPas bis 6000 mPas hergestellt werden können. Unter Salzen sind ein- oder mehrwerteige Alkalimetallverbindungen bzw. Erdalkalimetallverbindungen mit Halogenanionen zu verstehen. Besonders bevorzugt wird Natriumchlorid eingesetzt.

Es ist erfindungsgemäß von Vorteil ein oder mehrere Alkylisalze bzw. Erdalkylisalze in einer Konzentration von 0,1 bis 3 Gewichts-%, bezogen auf das Gesamtgewicht der Zubereitung einzusetzen.

Bei mit Salzen schwerverdickbaren Zusammensetzungen ist es erfindungsgemäß von Vorteil, wenn in den erfindungsgemäßen Zubereitungen Verdicker eingesetzt werden. Diese können beispielsweise vorteilhaft aus weiteren Verbindungen der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form als Verdicker verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Noveon (Carbopol 980, 981, 1382, 5984, 2984, ETD 2001, ETD 2020, ETD 2050 oder Pemulen TR1 & TR2).

Unter den Polymeren befinden sich z.B. Polyacrylamide (Seppigel 305), Polyvinylalkohole, PVP, PVP / VA Copolymere, Polyglycole.

Es ist erfindungsgemäß von Vorteil ein oder mehrere Verdicker in einer Konzentration von 0,1 bis 5 Gewichts-%, bezogen auf das Gesamtgewicht der Zubereitung einzusetzen. Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Die wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Ultrez 10, jeweils einzeln oder in Kombination.

Die kosmetischen Zubereitungen gemäß der Erfindung können kosmetische Hilfsstoffe enthalten, wie sie üblicherweise in solchen Zubereitungen verwendet werden, z. B. Konservierungshelfer, Komplexbildner, Antioxidantien, Puffer, Lösungsvermittler, Dispergiermittel, Bakterizide, Parfüme, Substanzen zum Verhindern oder Steigern des Schäumens, Farbstoffe, Pigmente, die eine färbende Wirkung haben, Verdickungsmittel, Perlglanzpigmente, weichmachende, anfeuchtende und/oder feuchhaltende Substanzen, Fette, Öle, Wachse oder andere übliche Bestandteile einer kosmetischen oder dermatologischen Formulierung wie Emulgatoren, Alkohole, Polyole, Polymere, Schaumstabilisatoren, Elektrolyte, organische Lösungsmittel oder Silikonderivate.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Antischuppenwirkstoffe, Pflanzenextrakte, Vitamine, Wirkstoffe.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Calcium-Vitamin-Komplexen erfindungsgemäß besonders vorteilhaft.

Ferner ist es im Sinne der vorliegenden Erfindung, der erfindungsgemäßen Zubereitung Perlglanzpigmente, Glimmer und/oder Effektpigmente zuzusetzen, um die Zubereitung optisch attraktiver zu gestalten.

Die erfindungsgemäßen Zubereitungen stellen in jeglicher Hinsicht überaus befriedigende Präparate dar, welche nicht auf die in dieser Schrift erwähnte Rohstoffauswahl begrenzt sind.

Es ist erfindungsgemäß vorteilhaft, wenn die erfindungsgemäße kosmetische Zubereitung in einer Flasche, Quetschflasche, Pumpspray-, oder Aerosoldose aufbewahrt und aus dieser heraus angewendet werden. Entsprechend sind auch Flaschen, Quetschflaschen, Pumpspray-, oder Aerosoldosen, welche eine erfindungsgemäße Zubereitung enthalten, erfindungsgemäß.

Die erfindungsgemäßen Zubereitungen werden vorteilhaft zur Pflege des Haars, insbesondere des Kopfhaares, eingesetzt.

Erfindungsgemäß ist auch die Verwendung der erfindungsgemäßen kosmetischen Zubereitung als Haarshampoo und zum Konditionieren des Haares.

Die folgenden Beispiele, in welchen Waschpräparate zur Haarpflege beschrieben werden, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispielrezepturen

| | **1** | **2** | **3** | **4** | | |
|---|---|---|---|---|---|---|
| Natrium Laurylethersulfat | 9 | 9 | 9 | 9 | | |
| Cocamidopropyl Betain | 4 | 4 | 4 | 4 | | |
| Zitronensäure-alkylpolyglykolester-Sulfosuccinat Dinatriumsalz | 3 | 3 | 3 | 3 | | |
| N-Kokosfettsäureamidoethyl-N-hydroxyethylglycinat Natriumsalz | - | - | - | - | | |
| Natrium Carbomer | - | - | - | 0,2 | | |
| Polyquaternium-10 (Ucare Polymer JR 400 von Amerchol) | 0,3 | 0,2 | 0,2 | 0,3 | | |
| Guar Hydroxypropyl Trimonium Chlorid (Jaguar Excel von Rhodia) | 0,1 | 0,1 | 0,2 | 0,1 | | |
| PEG-3 Distearate (CUTINA TS von Cognis) | 1,5 | - | - | - | | |
| Glycol Distearat + Glycerin + Laureth-4 + Cocamidopropyl Betain (Euperlan PK 3000 OK von Cognis) | - | 4 | - | - | | |
| Glycol Distearat + Laureth-4 + Cocamidopropyl Betain (Euperlan PK 4000 von Cognis) | - | - | 4 | - | | |
| Glycol Distearat + Coco Glucoside + Glyceryl Oleate + Glyceryl Stearat (Lamesoft TM Benz von Cognis) | - | - | - | 4 | | |
| PEG-40 hydriertes Rizinusöl | 0,2 | 0,2 | 0,2 | 0,2 | | |
| Natriumchlorid | 0,2 | 0,2 | 0,15 | 0,2 | | |
| Natrium Salicylat | 0,4 | 0,4 | 0,4 | 0,4 | | |
| Natrium Benzoat | 0,4 | 0,4 | 0,4 | 0,4 | | |
| Parfüm | q.s. | q.s. | q.s. | q.s. | | |
| Wasser | ad 100 | ad 100 | ad 100 | ad 100 | | |

| | **5** | **6** | **7** | **8** | **9** | **10** |
|---|---|---|---|---|---|---|
| Natrium Laurylethersulfat | 4 | 9 | 9 | 9 | 9 | 9 |
| Natrium Myristylethersulfat | 5 | - | - | - | - | - |
| Cocamidopropyl Betain | 4 | 4 | 4 | 4 | 4 | 5,0 |
| Zitronensäure-alkylpolyglykolester-Sulfosuccinat Dinatriumsalz | 2 | 3 | - | 3 | 3 | - |
| Dinatriumlaurylsulfosuccinat | - | - | 2 | - | - | - |
| Xanthan Gum | - | - | - | 0,1 | - | - |
| Natrium Carbomer | - | - | - | 0,2 | - | - |
| Polyquatemium-10 (Ucare Polymer JR 400 von Amerchol) | 0,3 | 0,2 | 0,2 | 0,3 | 0,3 | 0,3 |
| Guar Hydroxypropyl Trimonium Chlorid (Jaguar Excel von Rhodia) | 0,1 | 0,1 | 0,2 | 0,1 | 0,1 | 0,1 |
| PEG-3 Distearate (CUTINA TS von Coqnis) | 1,5 | 1,5 | 2,0 | 1,0 | - | - |
| Styrol/Acrylat Copolymere (Acusol OP 301 von Rohm & Haas) | - | - | - | - | 0,5 | 0,5 |
| Glycol Distearat + Coco Glucoside + Glyceryl Oleate + Glyceryl Stearat (Lamesoft TM Benz von Cognis) | - | - | - | 4 | - | - |
| PEG-40 hydriertes Rizinusöl | 0,2 | 0,2 | 0,2 | 0,2 | 0,2 | 0,2 |
| Natriumchlorid | 0,2 | 0,2 | 0,15 | 0,2 | 0,2 | 0,2 |
| Natrium Salicylat | 0,4 | 0,4 | 0,4 | 0,4 | 0,4 | 0,4 |
| Natrium Benzoat | 0,4 | 0,4 | 0,4 | 0,4 | 0,4 | 0,4 |
| Parfüm | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Zubereitung enthaltend
a) ein oder mehrere Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen,
b) ein oder mehrere Polymere, welche gebildet werden aus quaternisierten Ammoniumsalz der Hydroxypropylcellulose die mit einem Trimethylammonium substituierten Epoxid modifiziert sind (INCI: Polyquaternium-10) und ein mittleres Molekulargewicht von 200000 bis 1000000 und eine Ladungsdichte von 0,8 bis 1,8 meq/g aufweisen,
c) Natriumlaurylethersulfat,
d) Trübungsmittel/Perlglänze gewählt aus PEG-3 Distearat und/oder eine Kombination aus Glycoldistearat, Glycerin, Laureth-4 und Cocamidopropylbetain und/oder eine Kombination aus Glycoldistearat, Cocosglucosiden, Glyceryloleat und Glycerylstearat und/oder Styrol/Acrylat Copolymere neben gegebenenfalls weiteren kosmetischen Wirk-, Hilfs- und Zusatzstoffen.

2. Kosmetische Zubereitung nach Anspruch 1, **dadurch gekennzeichnet, dass** sie enthält
a) ein oder mehrere Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen, in einer Konzentration von 0,01 bis 3 Gewichts-%,
b) ein oder mehrere Polymere, welche gebildet werden aus quaternisierten Ammoniumsalz der Hydroxypropylcellulose, die mit einem Trimethylammonium substituierten Epoxid modifiziert sind (INCI: Polyquaternium-10) und ein mittleres Molekulargewicht von 200000 bis 1000000 und eine Ladungsdichte von 0,8 bis 1,8 meq/g aufweisen, in einer Konzentration von 0,01 bis 3 Gewichts-%,
c) Natriumlaurylethersulfat und ggf. ein oder mehrere Tenside in einer Konzentration von 1 bis 25 Gewichts-%, jeweils bezogen auf das Gesamtgewicht der Zubereitung
d) Trübungsmittel/Perlglänze gewählt aus PEG-3 Distearat und/oder eine Kombination aus Glycoldistearat, Glycerin, Laureth-4 und Cocamidopropylbetain und/oder eine. Kombination aus Glycoldistearat, Cocosglucosiden, Glyceryloleat und Glycerylstearat und/oder Styrol/Acrylat Copolymere neben gegebenenfalls weiteren kosmetischen Wirk-, Hilfs- und Zusatzstoffen.

3. Kosmetische Zubereitung nach einem der Ansprüche 1 bis 2, **dadurch gekennzeichnet, dass** das Verhältnis der Komponenten a) und b) von 0.1:10 bis 10:0,1 beträgt.

4. Kosmetische Zubereitung nach einem der Ansprüche 1 bis 3, **dadurch gekennzeichnet, dass** als weitere Tenside anionische, amphotere und/oder nichtionische Tenside wie Cocoamidopropylbetain und/oder Zitronensäure-alkylpolyglykolester-Sulfosuccinat Dinatriumsalze und/oder N-Kokosfettsäureamidoethyl-N-hydroxyethylglycinat Natriumsalze eingesetzt werden.

5. Kosmetische Zubereitung nach einem der Ansprüche 1 bis 4, **dadurch gekennzeichnet, dass** sie als weitere Bestandteile Verdicker enthält.

6. Verwendung einer kosmetische Zubereitung nach einem der vorhergehenden Ansprüche als Haarshampoo.

7. Verwendung einer kosmetischen Zubereitung nach einem der vorhergehenden Ansprüche zum Konditionieren des Haares.

## Claims

1. Cosmetic preparation comprising
a) one or more guar hydroxypropyltrimethylammonium chlorides which belong to the class of the galactomannan 2-hydroxypropyltrimethylammonium chloride ethers, have an average molecular weight of 600 000 to 2 000 000 and have a charge density of 0.4 to 1.0 meq/g,
b) one or more polymers which are formed from quaternized ammonium salts of hydroxypropylcellulose which have been modified with a trimethylammonium-substituted epoxide (INCI: Polyquaternium-10) and have an average molecular weight of 200 000 to 1 000 000 and a charge density of 0.8 to 1.8 meq/g,
c) sodium lauryl ether sulphate,
d) opacifiers/pearlescents selected from PEG-3 distearate and/or a combination of glycol distearate, glycerol, laureth-4 and cocamidopropylbetaine and/or a combination of glycol distearate, cocoglucosides, glyceryl oleate and glyceryl stearate and/or styrene/acrylate copolymers alongside optionally further cosmetic active ingredients, auxiliaries and additives.

2. Cosmetic preparation according to Claim 1, **characterized in that** it comprises
a) one or more guar hydroxypropyltrimethylammonium chlorides which belong to the class of galactomannan 2-hydroxypropyltrimethylammonium chloride ethers, have an average molecular weight of 600 000 to 2 000 000 and have a charge density of 0.4 to 1.0 meq/g, in a concentration of 0.01 to 3% by weight,
b) one or more polymers which are formed from quaternized ammonium salts of hydroxypropylcellulose which have been modified with a trimethylammonium-substituted epoxide (INCI: Polyquaternium-10) and have an average molecular weight of from 200 000 to 1 000 000 and a charge density of 0.8 to 1.8 meq/g, in a concentration of 0.01 to 3% by weight,
c) sodium lauryl ether sulphate and optionally one or more surfactants in a concentration of 1 to 25% by weight, in each case based on the total weight of the preparation
d) opacifiers/pearlescents selected from PEG-3 distearate and/or a combination of glycol distearate, glycerol, laureth-4 and cocamidopropylbetaine and/or a combination of glycol distearate, cocoglucosides, glyceryl oleate and glyceryl stearate and/or styrene/acrylate copolymers alongside optionally further cosmetic active ingredients, auxiliaries and additives.

3. Cosmetic preparation according to one of Claims 1 to 2, **characterized in that** the ratio of components a) and b) is from 0.1:10 to 10:0.1.

4. Cosmetic preparation according to one of Claims 1 to 3, **characterized in that** the further surfactants used are anionic, amphoteric and/or nonionic surfactants such as cocamidopropylbetaine and/or citric acid alkyl polyglycol ester sulphosuccinate disodium salts and/or N-coconut-fatty acid amidoethyl-N-hydroxyethylglycinate sodium salts.

5. Cosmetic preparation according to one of Claims 1 to 4, **characterized in that** it comprises thickeners as further constituents.

6. Use of a cosmetic preparation according to one of the preceding claims as hair shampoo.

7. Use of a cosmetic preparation according to one of the preceding claims for conditioning the hair.

## Revendications

1. Préparation cosmétique contenant
a) un ou plusieurs chlorures d'hydroxypropyltriméthyl-ammonium-guar qui appartiennent à la classe des éthers de chlorures de 2-hydroxypropyltriméthylammonium-galactomannanes, ont une masse moléculaire moyenne de 600 000 à 2 000 000 et présentent une densité de charge de 0,4 à 1,0 mEq/g,
b) un ou plusieurs polymères qui sont formés à partir de sels d'ammonium quaternisés de l'hydroxypropylcellulose qui sont modifiés avec un époxyde substitué par triméthylammonium (INCI : polyquaternium-10) et présentent une masse moléculaire moyenne de 200 000 à 1 000 000 et une densité de charge de 0,8 à 1,8 mEq/g,
c) du lauryléthersulfate de sodium,
d) des opacifiants/agents nacrants choisis parmi le PEG-3 distéarate et/ou une association de distéarate de glycol, glycérol, laureth-4 et cocosamidopropylbétaïne et/ou une association de distéarate de glycol, glucosides de coco, oléate de glycéryle et stéarate de glycéryle et/ou copolymères styrène/acrylate en plus éventuellement d'autres actifs, adjuvants et additifs cosmétiques.

2. Préparation cosmétique selon la revendication 1, **caractérisée en ce qu'**elle contient
a) un ou plusieurs chlorures d'hydroxypropyltriméthylammonium-guar qui appartiennent à la classe des éthers de chlorures de 2-hydroxypropyltriméthylammonium-galactomannanes, ont une masse moléculaire moyenne de 600 000 à 2 000 000 et présentent une densité de charge de 0,4 à 1,0 mEq/g, à une concentration de 0,01 à 3 % en poids,
b) un ou plusieurs polymères qui sont formés à partir de sels d'ammonium quaternisés de l'hydroxypropylcellulose qui sont modifiés avec un époxyde substitué par triméthylammonium (INCI : polyquaternium-10) et présentent une masse moléculaire moyenne de 200 000 à 1 000 000 et une densité de charge de 0,8 à 1,8 mEq/g, à une concentration de 0,01 à 3 % en poids,
c) du lauryléthersulfate de sodium et éventuellement un ou plusieurs tensioactifs à une concentration de 1 à 25 % en poids, chaque fois par rapport au poids total de la préparation,
d) des opacifiants/agents nacrants choisis parmi le PEG-3 distéarate et/ou une association de distéarate de glycol, glycérol, laureth-4 et cocosamidopropylbétaïne et/ou une association de distéarate de glycol, glucosides de coco, oléate de glycéryle et stéarate de glycéryle et/ou copolymères styrène/acrylate en plus éventuellement d'autres actifs, adjuvants et additifs cosmétiques.

3. Préparation cosmétique selon la revendication 1 ou 2, **caractérisée en ce que** le rapport des composants a) et b) vaut de 0,1:10 à 10:0,1.

4. Préparation cosmétique selon l'une quelconque des revendications 1 à 3, **caractérisée en ce qu'**on utilise comme autres tensioactifs des tensioactifs anioniques, amphotères et/ou non ioniques, tels que la cocosamidopropylbétaïne et/ou des sels disodiques d'alkylpolyglycolester d'acide citrique-sulfosuccinate et/ou des sels sodiques de glycinate de N-amidoéthyl d'acide gras de coco-N-hydroxyéthyle.

5. Préparation cosmétique selon l'une quelconque des revendications 1 à 4, **caractérisée en ce qu'**elle contient comme autres composants des épaississants.

6. Utilisation d'une préparation cosmétique selon l'une quelconque des revendications précédentes, en tant que shampooing capillaire.

7. Utilisation d'une préparation cosmétique selon l'une quelconque des revendications précédentes, pour le conditionnement du cheveu.
